Europäisches Patentamt

European Patent Office

Office européen des brevets

(19)

(11) Publication number: **0 116 744**
A1

(12) EUROPEAN PATENT APPLICATION

(21) Application number: 83305331.7

(22) Date of filing: 13.09.83

(51) Int. Cl.³: **A 61 J 3/07**

(30) Priority: 20.12.82 US 451580

(43) Date of publication of application:
29.08.84 Bulletin 84/35

(84) Designated Contracting States:
AT BE CH DE FR GB IT LI LU NL SE

(71) Applicant: WARNER-LAMBERT COMPANY
201 Tabor Road
Morris Plains New Jersey 07950(US)

(72) Inventor: Wittwer, Fritz
Bündtenstrasse 11
CH-4411 Lupsingen(CH)

(74) Representative: Jones, Michael Raymond et al,
HASELTINE LAKE & CO. 28 Southampton Buildings
Chancery Lane
London WC2A 1AT(GB)

(54) Apparatus for and method of sealing capsules.

(57) A method is disclosed for the sealing of gelatin or other hydrophilic polymer capsules having hard shell coaxially alignable cap and body parts which overlap when telescopically joined. Also described are apparatus and sealing fluids to seal the capsules.

The method comprises sealing the cap and body parts by contact with a sealing fluid, in the region of overlap of the cap and body parts.

An apparatus for performing the method is also disclosed. The apparatus comprises: a funnel (5) for receiving the capsules (4) and applying them to a conveyor (14); wetting means (6,7) for applying sealing fluid to the capsules; and drying means (9) for removing excess fluid from the capsules.

EP 0 116 744 A1

*FIG. 1*

0116744

-1-

APPARATUS FOR AND METHOD OF SEALING CAPSULES

This invention relates to a method of and apparatus for sealing capsules, using a sealing fluid, and to a capsule formed thereby.

The capsules sealed by the method and apparatus according to the present invention are hard shell, telescopically joinec capsules, having coaxial cap and body parts. The capsules may be made of gelatin and/or of other materials whose properties are pharmaceutically acceptable.

In this application, when the term "gelatin" is used it is also understood to include gelatin and/or other hydrophilic polymers.

Also contemplated by the present invention is the sealing of so-called foam capsules which are disclosed in our European Patent Application No. 83304741.8.

Sealing fluids for use in the sealing of capsules are also disclosed in: United States Patent No. 3,071,513 which discloses a sealing fluid comprising a dispersion of an air-drying hydrophilic, film-forming polymer in an organic solvent, the application of the sealing fluid being by way of dipping the capsules; and United States Patent No. 3,159,546 which discloses a liquid sealant consisting of three components containing by weight from about 1 to 4.5 parts, preferably 3 to 4.5 parts, of acetone, from about 1.25 to 2 parts, and preferably 1.5 to 2 parts, of water and from about 0.75 to 2.25 parts, and preferably about 0.75 of a part, of ethyl acetate, the application of the liquid solvent being by drop application.

Hard shell gelatin capsules have a disadvantage when compared with other forms of dosage of medicaments, in that the cap and body parts can be opened and rejoined without the disruption becoming externally visible and without apparent evidence of tampering. Therefore, the consumer has no real guarantee that the contents of a capsule have not been tampered with.

Telescopically joined, hard shell gelatin capsules have an overlap of the cap side wall over the body side wall which can be designed to impede gripping and withdrawl of the body, thereby making separation difficult.

According to a first aspect of the present invention there is provided a method of sealing a capsule made from gelatin and/or another hydrophilic polymer, having hard shell coaxially alignable cap and body parts which overlap when telescopically joined, which method comprises the steps of:

(i) causing a sealing fluid to make contact with the region of overlap of the cap and body parts; and

(ii) removing excess sealing fluid from the capsule.

The sealing fluid may be applied by: spraying the capsule with the sealing fluid; exposing the capsule to the sealing fluid in a vacuum-steam chamber; or dipping the capsule in the sealing fluid, which is directed to the overlap region and between the parts by capillary action.

The sealing fluid may comprise one or more of:- an organic solvent which depresses the melting point of gelatin or other hydrophilic polymer and which has a solubility parameter in the range of between about 10 to about 23.4 (calories per cubic centimetre) $^{\frac{1}{2}}$ ; an aqueous solution of a salt, or the corresponding acids and/or bases of the salt, having cations and anions which depress the melting point of gelatin or other hydrophilic polymer; an aqueous solution of an organic solvent, which depresses the melting point of gelatin and which has a solubility parameter in the range of between about 10 and about 23.4 (calories per cubic centrimetre) $^{\frac{1}{2}}$ , and of a salt, or the corresponding acids and/or bases of the salt, having cations and anions of the salt depressing the melting point of the gelatin; or a vapour of a liquid sealing fluid.

According to a second aspect of the present invention there is provided a hard shell gelatin capsule comprising cap and body parts which have been telescopically joined with a wall of one part overlapping a wall of the other part, and sealed by a method according to the first aspect of the present invention.

A capsule according to the second aspect of the present invention may be filled with one or more of the following: powder; a paste; tablets, pellets, granules or micro-capsules; a liquid; and a solid and a liquid.

According to a third aspect of the present invention there is provided an apparatus for sealing hard shell gelatin capsules having coaxial cap and body parts which overlap when telescopically joined, the apparatus comprising:

a conveyor having container means associated therewith for receiving the capsules;

means for exposing the capsules to a sealing fluid; and

drying means for removing sealing fluid from the surface of the capsule.

The present invention, by using a sealing fluid applied to the overlap of the cap side wall over the body side wall, makes the capsule tamper-resistant by spot or complete sealing of the overlap of the capsule parts. With the use of a complete sealing, the capsules are also tight against leakage of liquid contents.

For a better understanding of the present invention, and to show how the same may be carried into effect, reference will now be made, by way of example, to the accompanying drawings in which:-

Figure 1 shows a schematic of a continuous conveyor 1 having net or wire mesh baskets 2;

Figure 2 shows an embodiment of the present invention wherein filled and telescopically joined capsules 4 are oriented and held with the cap part upright while the overlap of the cap and body part side walls of each capsule 4 are contacted by the sealing fluid 6 within a dipping tank 7;

Figure 3 shows another embodiment of the present invention wherein the filled and telescopically joined capsules 4 are conveyed through a spray chamber 12 wherein sealing fluid 6 is sprayed by a nozzle 13 so as to contact the sealing fluid 6 with the overlap of the cap and body part side walls of each capsule 4;

Figure 4 shows another embodiment of the present invention wherein the sealing fluid 6, or a vapour thereof, is sprayed before the capsule 4 is telescopically joined, by a spray nozzle 13 which sprays the sealing fluid 6, or a vapour thereof, into the open end 15 and/or onto the inside of the side walls 16 of the cap part of the capsule 4; and

Figure 5 shows another embodiment of the present invention wherein the sealing fluid 6 is sprayed after the capsule 4 is telescopically joined.

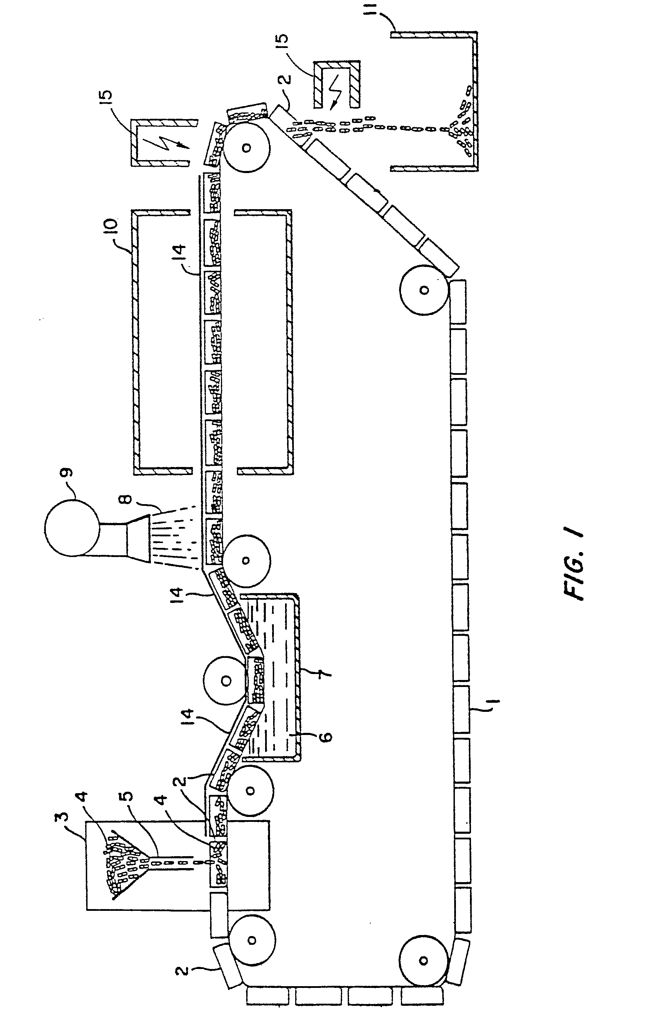

In the embodiment of Figure 1, a capsule filling machine 3 ejects filled and telescopically joined hard shell gelatin capsules 4 through a funnel 5 into the mesh baskets 2 which pass beneath the funnel 5. The capsules 4 are randomly oriented in the mesh baskets 2, which capsules 4 are then dipped into a sealing fluid 6 contained in a dipping tank 7. It is essential that the overlap of the cap and body side walls of each capsule 4 come into contact with the sealing fluid 6. Thereafter, the capsules 4 are conveyed through a drying stream of conditioned air 8 from a blower 9 located above or below the capsules 4 in order to remove excess sealing fluid 6 from the surface of the capsules 4 so as to avoid deformation and sticking together of the capsules 4. However, the sealing fluid is removed only from the surface, and not from within the overlapping seal of each capsule. The surface dried capsules are then fully dried by a dryer 10 which may be a kiln; an oven; a tumbler dryer; etc. Thereafter, the capsules 4 are conveyed to and ejected into a capsule container 11 for further processing and shipment. A cover 14 is provided over the mesh baskets

-6-

2 to prevent floating-away or blowing-away of the capsules 4 during processing between the funnel 5 and the dryer 10.

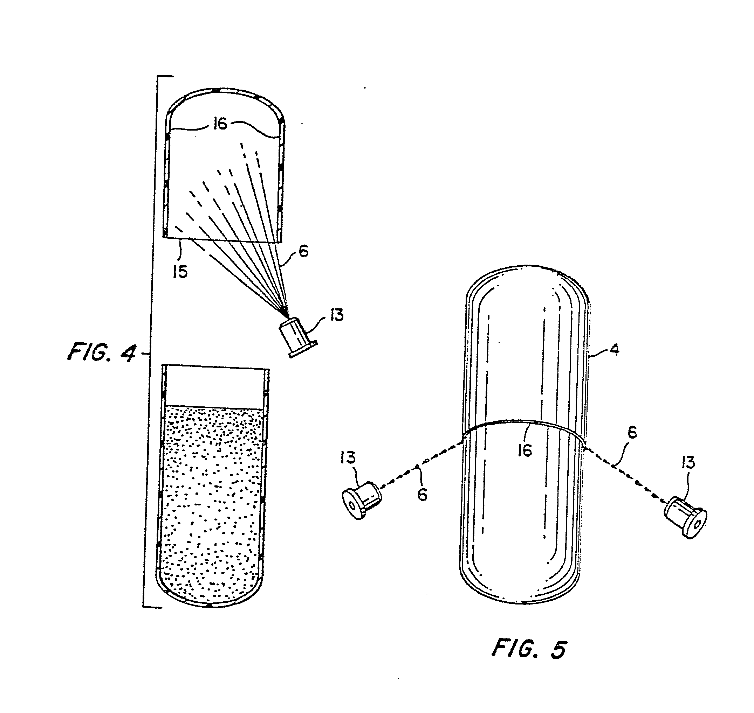

In the embodiment shown in Figure 4, the sealing fluid or a vapour thereof may be sprayed into the open end and or onto the inside of the side walls of the cap part of the capsule 4. This embodiment of the present invention may be connected to a capsule filling machine.

In the embodiment shown in Figure 5, the capsule 4 is sealed by spraying the sealing fluid 6 or a vapour thereof onto the seam 16 of the overlap of the cap and body part side walls of the capsule 4. This embodiment of the present invention may be connected to a capsule sealing machine or used separately.

The sealing of capsules in the present invention is accomplished as follows:

The sealing fluid is evenly distributed between the overlap of the cap and body side walls of the gelatin capsule by capillary effect. This effect is achieved when the contact angle between a drop of the sealing fluid and the gelatin film is small, e.g. if the wettability of the gelatin film is high, the contact angle can be reduced by the addition of surfactants.

The mechanism of the capillary effect is described by Walter J. Moore in Physical Chemistry, 4th Edition, pages 479-481, Longman Edition, London, England, (1978) as follows: "Whether a liquid rises in a glass capillary depends on the relative magnitude of the forces of adhesion between the liquid molecules themselves, and the forces of adhesion between the liquid and the walls of the tube. These forces determine the contact angle, which the liquid makes with the tube walls. If this angle is less than

0116744

-7-

90 degree, the liquid is said to wet the surface and a concave meniscus is formed".

The wettability of gelatin films is measured as "adhesional wetting" where a liquid not originally in contact with a substrate makes contact with that substrate and adheres to it.

The contact angles between gelatin films and solvents were measured for a number of sealing fluids of the present invention by use of a microscope fitted with a goniometer eyepiece.

The tests were performed on a gelatin film whereby the contact angle was measured 20 seconds after depositing a drop of sealing fluid on the gelatin film. The following Table shows contact angles of sealing fluids of the present invnetion:

## TABLE I

| Sealing Fluids | Mean Contact Angles |
|---|---|
| &mdash; Water | $83° \pm 6°$ |
| &mdash; 75% aqueous ethyl alcohol solution | $3.5° \pm 1°$ |
| &mdash; 75% ethyl alcohol solution mixed with an aqueous solution of 0.5M $CaCl_2$ and 1M KI | near to 0° (not detectable) |
| — 90% aqueous methanol solution | near to 0° (not detectable) |

The sealing fluid swells the gelatin between overlap of the cap side walls over the body side walls of the capsule.

The melting point of the swollen gelatin is depressed by the sealing fluid below room temperature so that the sealing of the overlap occurs at about room temperature. This step of the method is a denaturation process of the gelatin reflecting a peptization or gelatination as stated by D. J. Lloyd and M. Garrod, Transactions Faraday Society 44, 441 (1948).

The sealing fluids used in the present invention contain a considerable amount of water. A depression of the melting point of gelatin is a necessary effect for the present invention. This effect can be achieved by the following groups of sealing fluids:

1.  Organic Solvents

Sealing fluids of effective organic solvents depressing the melting point of gelatin; having a solubility parameter between about 10 to about 23.4; and being sufficiently miscible with water are given in TABLE 2 below. (References:

— J. Brandrupp and E. H. Immugut, Polymer Handbook, 1st Edition, pages IV 356-358, John Wiley N.Y. (1966)

— J. Bello, H. C. A. Riese, J. R. Vinograck, J. Phys. Chem 60, (1956)

1/2

| S (cal/cc) | Organic Solvent |
|---|---|
| 10.0 | amyl alcohol (iso) |
| 10.0 | carbon disulfide |
| 10.0 | dichlorobenzene (ortho) |
| 10.0 | diethyl phthalate |
| 10.0 | dimethyll-2,2-butanedfol-1.3 |
| 10.0 | dioxane-1,4 |
| 10.0 | dipropylene glygol |
| 10.0 | ethylamine |
| 10.0 | ethylene glycol diacetate |
| 10.0 | ethyl lactate |
| 10.0 | methyl isobutyl carbinol |
| 10.0 | nitrobenzene |
| 10.0 | propionic anhydride |
| 10.1 | acetic acid |
| 10.1 | caprolactone |
| 10.1 | dibromoethylene-1,2 |
| 10.1 | propylene glycol methyl ether |
| 10.2 | cresol (meta) |
| 10.2 | diethylene glycol monoethyl ether |
| 10.2 | dioxolane-1,3 |
| 10.2 | methyl formate |
| 10.2 | methyl iodine |
| 10.3 | acetaldehyde |
| 10.3 | acetic anhydride |
| 10.3 | aniline |
| 10.3 | butyric acid (iso) |
| 10.3 | hexanediol-2,5 |
| 10.3 | methyl-2-pentanediol-1,3 |
| 10.3 | nitro-1-propane |
| 10.3 | octyl alcohol (normal) |
| 10.4 | cyclopentanone |
| 10.4 | dibromoethane-1,2 |
| 10.5 | acrylonitrile |
| 10.5 | butyl alcohol (iso) |
| 10.5 | butyric acid (normal) |
| 10.5 | butyronitrile |

| | |
|---|---|
| 10.5 | butyronitrile |
| 10.5 | ethyl-2-butanol-1 |
| 10.5 | ethylene glycol monoethyl ether |
| 10.5 | hexamethylphosphoramide |
| 10.5 | methyl benzoate |
| 10.6 | bromonapthalene |
| 10.6 | butyl alcohol (tert.) |
| 10.6 | diethylformamide (N,N) |
| 10.6 | heptyl alcohol (normal) |
| 10.6 | methyl salicylate |
| 10.7 | dimethyl phthalate |
| 10.7 | hexyl alcohol (normal) |
| 10.7 | pyridine |
| 10.7 | triethylene glycol |
| 10.8 | butyl alcohol (secondary) |
| 10.8 | dimethylacetamide (N,N) |
| 10.8 | pentanediol-2,4 |
| 10.8 | propionitrile |
| 10.8 | quinoline |
| 10.9 | amyl alcohol (normal) |
| 11.0 | cyclobutanedione |
| 11.0 | dichloroacetic acid |
| 11.0 | dimethyl malonate |
| 11.0 | dimethyl oxalate |
| 11.0 | ethyl cyanoacetate |
| 11.0 | neopentyl glycol |
| 11.1 | butanediol-2,3 |
| 11.1 | ethylene oxide |
| 11.1 | nitroethane |
| 11.2 | acetylpiperidine (N) |
| 11.2 | dimethyl-2,2-butanediol-1,2 (Isobutylene glycol) |
| 11.2 | furfural |
| 11.2 | methacrylic acid |
| 11.2 | methylamine |
| 11.3 | dipropyl sulfone |
| 11.3 | methylpyrrolidone-2 (N) |

| 11.4 | acetylpyrrolidine (N) |
| 11.4 | butyl alcohol (normal) |
| 11.4 | cyclohexanol |
| 11.4 | ethylene glycol monomethyl ether |
| 11.4 | tetramethyloxamide |
| 11.5 | formylpiperidine (N) |
| 11.5 | pentanediol-1,5 |
| 11.5 | propyl alcohol (iso) |
| 11.6 | hacetylmorphaline (N) |
| 11.6 | butanediol-1,3 |
| 11.8 | allyl alcohol |
| 11.8 | methylene fodide |
| 11.9 | acetonitrile |
| 11.9 | propyl alcohol (normal) |
| 11.9 | Santicizer 8 |
| 12.0 | acrylic acid |
| 12.0 | dimethyl sulfoxide |
| 12.1 | benzyl alcohol |
| 12.1 | butanediol-1,4 |
| 12.1 | butylene-2,3 carbonate |
| 12.1 | diethylene glycol |
| 12.1 | dimethylformamide (N,N) |
| 12.1 | dimethyltetramethylene sulfone |
| 12.1 | formic acid |
| 12.1 | hydrogen cyanide |
| 12.2 | ethylene chlorohydrin |
| 12.3 | ethylacetamide (N) |
| 12.4 | diethyl sulfone |
| 12.4 | methylene glycolate |
| 12.5 | dimethyl phosphite |
| 12.5 | furfuryl alcohol |
| 12.5 | methyl proply sulfone |
| 12.6 | butyrolactone |
| 12.6 | chloracetonit |
| 12.6 | propylene gylcol |
| 12.7 | caprolactam (epsilon) |
| 12.7 | ethyl alcohol |

| 12.7 | nitromethane |
|---|---|
| 12.9 | methyltetramethylene sulfone |
| 13.0 | formylmorpholine (N) |
| 13.1 | dimethylnitroamine (N,N) |
| 13.3 | propiolactone |
| 13.3 | propylene-1,2 carbonate |
| 13.4 | methyl ethyl sulfone |
| 13.4 | pyrone (gamma) |
| 13.4 | tetramethylene sulfone |
| 13.6 | maleic anhydride |
| 13.6 | piperidone |
| 13.7 | diacetylpiperazine (N,N) |
| 13.9 | ethylformamide (N) |
| 14.5 | methanol |
| 14.5 | dimethyl sulfone |
| 14.6 | ethylene glycol |
| 14.6 | methylacetamide (N) |
| 14.7 | ethylene carbonate |
| 14.7 | pyrrolidone (alpha) |
| 15.1 | diformylpiperazine (N,N) |
| 15.4 | succinic anhydride |
| 16.1 | methylformamide (N) |
| 16.3 | ammonia |
| 16.3 | glycerol |
| 19.2 | formamide |
| 23.4 | water |

The above organic solvents with a solubility parameter below about 10, which are miscible with water, can be used at low concentrations in combination with solvents having a solubility parameter above about 10 S(cal/cc).

For the sealing of pharmaceutical gelatin capsules, only pharmaceutically accepted organic solvents are used.

2.   Solutions of Salts

Sealing fluids of an aqueous solution of salts or an aqueous organic solution (Organic solvents from TABLE 2 above) of salts are effective for depressing the melting point of gelatin.  The effect of cations and anions of these salts is to depress the melting point of gelatin, as stated by K. H. Gustavson, The Chemistry and Reactivity of Collagen, Academic Press, N.Y. (1956) and may be explained as follows:

a) Cations like Ca$^{++}$ and Al$^{+++}$ are extremely efficient if their share is high and their radius small, which yields a strong polarization according to the Hofmeisters series.

b) Anions like SCN$^-$ and I$^-$ must possess a large electron cloud in order to have a strong polarization effect.

For the sealing of pharmaceutical gelatin capsules, only pharmaceutically acceptable salts are used.

Methods for the Application of Sealing Fluids to Capsules

Various methods were used for the application of the above solutions and organic solvents as sealing fluids for gelatin capsules:

1. Dipping of the entire capsules into a bath of the sealing fluid as shown in FIG. 1 for a time period of 1 to 5 seconds at a temperature range from between about 5 to 70°C followed by removing of the excess fluid from the capsule surface by a strong air jet. Thereafter, the capsules were dried

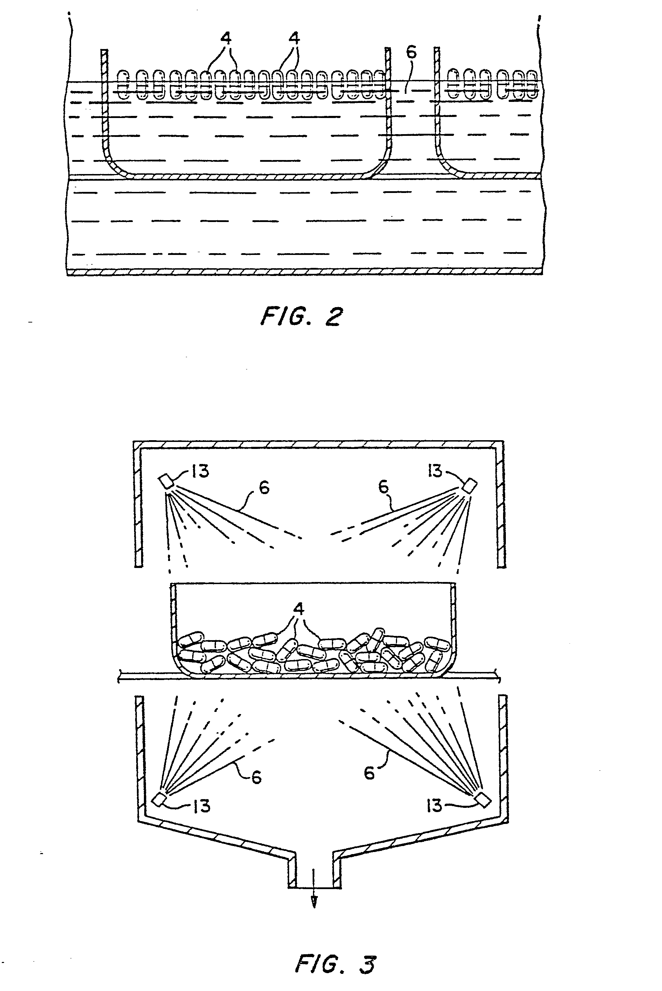

2. Dipping of the capsules in an upright position as shown in FIG. 2 so that the cap is on top and the overlap of the capsule is in contact with the sealing fluid. The sealing conditions were the same as in paragraph 1 above.

3. Spraying of the capsules with a sealing fluid as shown in FIG. 3. The sealing fluid was used at a temperature range between about 5 to 70°C. After spraying the excess fluid was removed from the capsule surface by a strong air jet (followed by capsule drying, if necessary to remove the sealing fluid).

4. Application of a sealing fluid to the capsules by using a high frequency pressure pulse jet nozzle as shown in FIGS 4 and 5 with an accurate monitoring of droplet delivery and deflection. Only minor drying was necessary. The sites of application of the sealing fluid were as follows:

- into and/or onto the open end of the cap part before capsule joining on a filling machine.

- onto the outside of the side walls of the open end of the body part before capsule joining on a filling machine.

- onto the overlap of the cap and body parts after capsule joining and filling.

5. Application of a steam of the sealing fluids by a jet nozzle as shown in FIGS. 4 and 5. Only minor drying was necessary. The sites of application were the same as in paragraph 4 above.

6. Capsule sealing by a steam of the sealing fluids selected was also accomplished by exposing the capsules in a combined steamvacuum chamber as disclosed in applicant's copending application, Serial Number 440,371 (PD-2988), the disclosure of which is incorporated herein by reference.

The sealing of capsules by the present invention can be used for hard shell gelatin capsules which have been telescopically joined and have the following contents:

    a.  Empty;

    b.  Powder;

    c.  Pastes;

    d.  Tablets, pellets, granules, microcapsules, etc/

    e.  Liquids (the sealing of the present invention was also successful in preventing leakage of oil from within the gelatin capsule); and

    f.  Liquids and solids.

For the sealing of gelatin capsules filled with oils, it was noted that an inverse capillary effect driving the oil between the overlap of the body and cap parts of the gelatin capsules may occur, especially when the filled gelatin capsules are held in a cap part down position. For rapeseed oil, having a viscosity of above about 90 centipoises, a contact angle between the gelatin film and the oil was measured which means that the capillary forces of oil are much lower than the capillary forces of the sealing fluids. Therefore, if the gelatin capsules are sealed within a few minutes after filling with an oil, the oily capsule content does not enter between the overlap of the body and the cap of the gelatin capsule. Hence, the capsules can be sealed by the sealing fluids.

If liquids or oils with low viscosities below about 90 centipoises and small contact angles are used, the following measures accomplished a complete sealing by the present invention:

- sealing the gelatin capsules within a few seconds after ejection from the filling machine.
- holding the gelatin capsule in an upright position with the cap part on top during the sealing process, as shown in FIG. 2.
- cooling the liquid contents prior to filling into the gelatin capsule in order to increase the viscosity and the contact angle between the gelatin film and the liquid.
- adding a thickening agent to the liquid contents prior to the filling process.

The best results were obtained without capsule deformation; with sealing fluids having a high degree of peptization, such as an aqueous solvent of 75% ethanol in water, and also with an aqueous solvent of 90% methanol in water.

-16-.

0116744

Example 1

Gelatin capsules, empty and filled with contents as described in b to e above, and telescopically joined, were sealed by the apparatus shown in FIG. 1 with a sealing fluid of 75% ethanol in water, at room temperature.

No sealing fluid was observed to enter the interior of the telescopically joined capsule during the dipping time of 1 to 5 seconds, and thereafter.

The sealed capsule parts could not be separated without destroying the capsules.

Example 2

20 empty gelatin capsules, size 4, were prepared so that the opening force could be measured as follows: Two 2 CM long needles fitted with small spherical heads (diameter 3 millimeters) were pierced through the cap and body parts so that both needle heads remained inside of the telescopically joined capsule. The capsules were filled with lactose and joined. 10 of these capsules were not sealed, but only stored overnight at 69% R.H. 10 capsules were sealed by a 3 sec. immersion in a solution of 75% ethanol and 25% water, at room temperature. After 30 minutes drying at 30°C. and 30% R.H., the capsules were stored overnight at 69% R.H. (the capsules having a a final humidity content of 15.5%). Comparative opening tests of unsealed and sealed capsules made done on an Instron Table Model 1130 fitted with a 10 lbs. tension load cell. The capsules were gripped at the outside parts of the above described needles. The opening force results were as follows:

|  | Unsealed Capsules | Sealed Capsules |
| --- | --- | --- |
| Mean force | 255 grams | 3,130 grams |
| Standard deviation | 58 grams | 1,460 grams |
| Results | Capsules opened; no damage | All capsules split at the side walls, but all seals remained intact. |

Thus, for sealed capsules, the resistance of the seal is higher than the resistance of the gelatin capsule side walls.

Example 3

100 capsules, size 2 (imprinted), were filled with lactose, joined and put on a sieve (diameter 20 CM), the latter being covered by another sieve. The capsules were sealed by a complete immersion of the sieves, during 3 seconds, at room temperature, into a sealing fluid of 75% ethanol and 25% water. Immediately after removal from the sealing fluid, the sieves were shaken for 10 seconds in order to remove the excess sealing fluid. Then a dry air flow with high velocity (18 M/Sec. Linear E.G. 150 M3/hour flow) was drawn through the sieves in order to dry the capsules very quickly. After one minute, the capsules were no longer sticky and could be stored in conventional packing boxes without any risk of sticking together. The capsules were not deformed. The print was only slightly faded.

Immediately after drying, opening test results on 30 capsules were:

    - 28 split and 2 opened, but with visible damage.

After four hours of supplementary drying at room temperature, 30 capsules were tested and the capsule parts could not be separated without destroying the capsules.

Example 4

Ten imprinted and joined gelatin capsules were sealed as in Example 3, with the following sealing fluids:

    A.  Methanol 90%, water 10%; for 3 sec.; at room temperature

    B.  Same as in A; except at 50°C.

    C.  Methanol 75%, water 25%; for 3 sec.; at room temperature

- 18 -

D. Same as in C; except at 50°C.     0116744

E. Methanol 60%, water 40%; for 3 sec.; at room
temperature

F. Same as in E; except at 50°C.

The capsules sealed with the sealing fluids in A
to D were dry after less than 1 minute; when dried with
an air flow of 30°C, 25% R.H., 150 M3/H. The capsules
sealed with the sealing fluids in E and F were dry
after less than 2 min.

No deformation was observed for all the capsules
sealed at room temperature. Slight deformation was
observed for for D; 10 capsules of 14 were observed as
deformed for B.

Complete sealing was observed in all cases. No
capsule could be opened without destruction of the
capsule.

Example 5

30 gelatin capsules, natural transparent, size 2,
were filled with rapeseed oil and telescopically joined.
Thereafter, the capsules were immersed for 3 seconds in
a solution of 60% methanol and 40% water, at room temperature. The capsules were then dried as described in
Example 2 above. The capsules were dry after 1 minute.
In addition, the capsules were stored for 3 minutes in
climatic cabinet at 30°C, 25 % R.H. When tested, all 30
capsules were found to be completely sealed and no oil
escaped during sealing operation. Also, there was no
deformation of the capsules. The capsule parts could
not be separated without destroying the capsules.

Example 6

100 gelatin capsules, natural transparent, size 2,
were filled with rapeseed oil (0.3g per capsule)
colored with Sudan red dye; having a viscosity of 90
CPS at 20°C. After joining, the capsules were put
between two sieves and immediately immersed in a
sealing fluid of 75% ethanol and 25% water, at room temperature, for 3 seconds. After removal from the sealing fluid, the sieves were shaken during 10 seconds and then a dry air (30°C., 25% RH) was blown with high velocity (150 M3/hour) onto the capsules. After one minute the capsules were no longer sticky and were stored without sticking together. The capsules were not deformed. After 30 minutes additional storage at 30°C and 25% RH, all 100 capsules were found to be completely sealed when strongly shaken or pressed between fingers. The capsules could not be separated without destroying the capsules.

While there have been described and illustrated several embodiments of the present invention, the scope and working range of the invention shall not be limited by examples given above. The invention comprises as well various changes and modifications which will occur to those skilled in the art.

It is intended in the appended claims to cover all such changes and modifications as fall within the true spirit and scope of the present invention.

0116744

CLAIMS:

1. A method of sealing a capsule made from gelatin and/or another hydrophilic polymer, having hard shell coaxially alignable cap and body parts which overlap when telescopically joined, which method comprises the steps of:

(i) causing a sealing fluid to make contact with the region of overlap of the cap and body parts; and

(ii) removing excess sealing fluid from the capsule.

2. A method according to Claim 1, wherein the sealing fluid is applied by: spraying the capsule with the sealing fluid; exposing the capsule to the sealing fluid in a vacuum-steam chamber; or dipping the capsule in the sealing fluid, which is directed to the overlap region and beteen the parts by capillary action.

3. A method according to Claim 1 or 2, wherein the sealing fluid is an organic solvent which depresses the melting point of the gelatin or other hydrophilic polymer and has a solubility parameter range of from 10 to 23.4 calories per cubic centimetre; an aqueous solution of said organic solvent and of a salt having cations and anions which depress the melting point of the gelatin or other hydrophilic polymer; an aqueous solution of a salt having cations and anions which depress the melting point of the gelatin or other hydrophilic polymer; or a vapour of a liquid sealing fluid.

4. A hardshell gelatin or other hydrophilic polymer capsule comprising cap and body parts which have been telecopically joined with a wall of one part overlapping a wall of the other part, and sealed by a method according to Claim 1 , 2 or 3.

5. A capsule as claimed in Claim 4, which has been filled with one or more of the following: powder; a paste; tablets; pellets granules or micro-capsules; a liquid; and a solid and a liquid.

6. An apparatus for sealing hard shell gelatin or other hydrophilic capsules having coaxial cap and body parts which overlap when telescopically joined, the apparatus comprising:

a conveyor having container means associated therewith for receiving the capsules;

means for exposing the capsules or parts therefof to a sealing fluid; and

drying means for removing sealing fluid from the surface of the capsules.

7. An apparatus as claimed in Claim 6, wherein the container means are net or wire mesh baskets.

8. An apparatus as claimed in Claim 6 or 7, wherein the means for exposing the capsules to a sealing fluid is a dipping tank, a spray chamber, or a vacuum-steam chamber.

9. An apparatus as claimed in Claim 6, 7 or 8, wherein the drying means is a blower; a blower and a kiln; a blower and a tumbler dryer; or a blower and an oven.

FIG. I

FIG. 2

FIG. 3

FIG. 4

FIG. 5

European Patent Office

**EUROPEAN SEARCH REPORT**

**0116744**
Application number

EP 83 30 5331

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,X | US-A-3 071 513 (H.R. DE BOER et al.) <br> * Claims 1, 12, 16; column 4, lines 68-75; column 5, lines 1-3, 16-20, 46-53; column 6, lines 39-46, 74, 75; column 7, lines 1-8 * | 1 | A 61 J 3/07 |
| A | | 2-9 | |
| X | US-A-3 073 087 (E.R. SANDHAGE et al.) <br> * Claims 1, 2; column 1, lines 9-15, 72; column 2, lines 1-5; column 3, lines 24-29 * | 1 | |
| A | | 3 | **TECHNICAL FIELDS SEARCHED (Int. Cl. 3)** |
| D,A | US-A-3 159 546 (J.R. KANE) <br> * Column 1, lines 32-42, 62-66; column 2, lines 19-27 * | 1,4,5 | A 61 J 3/07 |
| A | DE-A-1 767 032 (RÖHM GMBH) <br><br> * Claim; page 4, lines 22, 23; page 6, lines 3-9 * | 1-3,6, 8 | |
| A | GB-A- 956 300 (ORGANON LABORATORIES LTD.) <br> * Claim 1; page 1, lines 9-14, 40-55 * | 1 | |
| | --- | -/- | |

The present search report has been drawn up for all claims

| Place of search | Date of completion of the search | Examiner |
|---|---|---|
| BERLIN | 14-03-1984 | CLOT P.F.J. |

European Patent Office

**EUROPEAN SEARCH REPORT**

**0116744**
Application number

EP 83 30 5331

| DOCUMENTS CONSIDERED TO BE RELEVANT | Page 2 |

| Category | Citation of document with indication, where appropriate, of relevant passages | Relevant to claim | CLASSIFICATION OF THE APPLICATION (Int. Cl. ³) |
|---|---|---|---|
| A | US-A-3 394 983 (M. GREIF et al.)<br>* Column 3, lines 57-70; figure 2 * | 6 | |

TECHNICAL FIELDS SEARCHED (Int. Cl. ³)

The present search report has been drawn up for all claims

| Place of search<br>BERLIN | Date of completion of the search<br>14-03-1984 | Examiner<br>CLOT P.F.J. |